(19) Europäisches Patentamt
European Patent Office
Office européen des brevets

(11) EP 2 597 159 B1

(12) **EUROPEAN PATENT SPECIFICATION**

(45) Date of publication and mention of the grant of the patent:
**29.01.2014 Bulletin 2014/05**

(51) Int Cl.:
***C12Q 1/68*** *(2006.01)*

(21) Application number: **11190186.4**

(22) Date of filing: **28.11.2011**

(54) **Method for identifying cows with mastitis by bulk genotyping of tank milk**

Verfahren zur Identifizierung von Kühen mit Mastitis durch die Bündelgenotypisierung von Milch in einem Tank

Procédé pour identifier des vaches souffrant de mastite par un génotypage en gros du lait en cuve

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**29.05.2013 Bulletin 2013/22**

(73) Proprietor: **Université de Liège**
**4031 Angleur (BE)**

(72) Inventors:
- **Georges, Michel**
  **4000 LIEGE (BE)**
- **Blard, Grégoire**
  **4000 LIEGE (BE)**
- **Coppieters, Wouter**
  **4000 LIEGE (BE)**

(56) References cited:
**WO-A1-2010/142301 WO-A2-2007/146280**

- **BARKEMA H W ET AL: "Incidence of Clinical Mastitis in Dairy Herds Grouped in Three Categories by Bulk Milk Somatic Cell Counts", JOURNAL OF DAIRY SCIENCE, AMERICAN DAIRY SCIENCE ASSOCIATION, US, vol. 81, no. 2, 1 February 1998 (1998-02-01), pages 411-419, XP027048084, ISSN: 0022-0302 [retrieved on 1998-02-01]**
- **HOMER N ET AL: "Resolving Individuals Contributing Trace Amounts of DNA to Highly Complex Mixtures Using High-Density SNP Genotyping Microarrays", PLOS GENETICS, PUBLIC LIBRARY OF SCIENCE, SAN FRANCISCO, CA, US, vol. 4, no. 8, 29 August 2008 (2008-08-29) , XP002549173, ISSN: 1553-7390, DOI: 10.1371/JOURNAL.PGEN.1000167**
- **DETILLEUX J ET AL: "Application of a Mixed Normal Mixture Model for the Estimation of Mastitis-Related Parameters", JOURNAL OF DAIRY SCIENCE, AMERICAN DAIRY SCIENCE ASSOCIATION, US, vol. 83, no. 10, 1 October 2000 (2000-10-01), pages 2341-2349, XP026993550, ISSN: 0022-0302 [retrieved on 2000-10-01]**
- **DETILLEUX J C: "A hidden Markov model to predict early mastitis from test-day somatic cell scores", ANIMAL, vol. 5, no. 2, February 2011 (2011-02), pages 175-181, XP002675737,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the invention

This invention relates to a method for the detection of animals that develop mastitis or subclinical mastitis, by analyzing a sample of milk obtained from the "milk tank" (in which the milk of the animals on the farm is collected and stored). The method includes obtaining a sample of milk from the tank, extracting DNA from the tank milk, genotyping this DNA for common DNA sequence polymorphisms such as Single Nucleotide Polymorphisms (SNPs), determining the proportion of each allele in the tank milk for all analyzed polymorphisms, and determining the contribution of DNA of each animal to the tank milk from these allelic proportions. As the amount of DNA contributed by each animal depends on the number of somatic cells in its milk - a proven indicator of mastitis - animals developing mastitis can be identified from the amount of DNA they have contributed to the tank milk. The method requires that the animals that contributed milk to the tank are individually genotyped for the same DNA sequence polymorphisms, a situation which is increasingly corresponding to reality.

### Description of the background art

Mastitis is generally regarded as the most important health issue in dairy farming. Costs to the farmer result from treatment, decreased milk yield and value, and culling. In the Netherlands, the annual cost of mastitis to farmers has been estimated at -80 Euros per cow per year (f.i. Hogeveen et al., 2011).

One often distinguishes clinical from subclinical mastitis. Mastitis is said to be clinical when resulting in either milk or udder anomalies that are manifest to the farmer. Subclinical mastitis is defined as the presence of microorganisms in combination with elevated somatic cell counts (SCC) in the milk. 200,000 cells/ml is often utilized as SCC cut-off value. Subclinical mastitis is costly on its own because of its negative impact on milk yield, which is decreasing log-linearly with SCC.

At present, the control of mastitis is primarily driven by the detection and treatment of clinical mastitis. More effective detection of subclinical mastitis would be a valuable addition to mastitis control. In most circumstances, SCC for individual cows are only measured periodically (typically every 4-6 weeks) precluding close monitoring of the udder health status of individual cows. Advanced versions of milking robots will precociously detect changes in conductivity or even SCC, yet automatic milking has been associated with a decrease rather than an improvement in udder health (f.i. Hovinen & Pyörälä, 2011). Thus novel approaches to monitor SCC of individual animals would be a benefit to the sector.

### Summary of the invention

In view of the above, we herein describe a method that allows for the determination of SCC for individual milking animals (and hence detection of cows with subclinical mastitis) by genotyping a sample of milk from the farm's milk tank for a panel of Single Nucleotide Polymorphisms (SNPs). The method assumes that individual genotypes for the same SNPs are available for all animals on the farm. As genomic selection is becoming common practice in dairy cattle, an increasing proportion of the cow population is being SNP genotyped. As an example, in the spring of 2011, more than 85,000 Holstein-Friesian animals had been genotyped with one of the available SNP arrays in the US alone. The majority of these were females, and their proportion was rapidly increasing with time. We anticipate that farms in which all cows will be SNP genotyped will be commonplace in the near future, and this will progressively extend to other domestic animals including sheep and goat. The SNP genotypes for the individual cows may either be real genotypes or genotypes determined in silico through the process of imputation.

Thus, the present invention relates in a first embodiment to a method for indirectly determining the number of somatic cells in the milk of individual cows, the method comprising the steps of:

a. obtaining a sample of milk from the farm's milk tank,
b. extracting DNA from said sample,
c. genotyping said DNA for a collection of DNA sequence polymorphisms,
d. quantifying the proportion of the different alleles for each of the said DNA sequence polymorphisms in said DNA,
e. estimating the proportion of DNA contributed by each cow on the farm to tank's milk,
f. estimating the somatic cell scores of each cow on the farm from said proportion of DNA contributed.

The term "milking cows" in accordance with the present invention encompasses all cattle or cattle breeds from the species Bos taurus, Bos indicus or hybrids thereof.

The term "sample" refers to a certain volume of milk that is collected from the "milk tank".

The term "milk tank" refers to containers that are used on farms to collect and store the milk from the lactating cows on the farm. Commercial milk tanks typically allow refrigeration and mixing of the collected milk.

The term "Extracting DNA" refers to methods to extract DNA from a biological sample, which are well-known in the art and can be applied in the present invention. Standard protocols for the isolation of genomic DNA are inter alia referred to in in Sambrook, J., Russell, D.W., Molecular Cloning: A Laboratory Manual, the third edition, Cold Spring Harbor Laboratory Press, Cold Spring Harbor, New York, 1.31-1.38, 2001 and Sharma, R.C., et al., A rapid procedure for isolation of RNA-free genomic DNA from mammalian cells, BioTechniques, 14, 176-178, 1993.

The term "DNA sequence polymorphisms" refers to segment of the genome that may differ in sequence between individuals. DNA sequence polymorphisms include (i) Single Nucleotide Polymorphisms (SNPs) that affect a single nucleotide position (and include transitions, transversions and single nucleotide insertions and deletions), (ii) microsatellites or simple tandem repeats (STRs) and minisatellites or variable number of tandem repeats (VNTRs) characterized by variable numbers of tandem copies of sequence motifs ranging from one to 100 base pairs in length, (iii) indels or insertion deletions of more than one nucleotide, and (iv) structural variants including Copy Number Variations (CNV). There are at present more than 10 million known SNPs for the bovine (http://www.ncbi.nlm.nih.gov/projects/SNP/) and many combinations of these could be included in a panel of DNA sequence polymorphisms selected to achieve the goal of the present invention. It is anticipated that similar collections of SNPs will soon become available for other domestic animal species including sheep and goat. As a reference genomic sequence is available for cattle and will soon become available for all the important domestic species including sheep and goat, hundreds of thousands of microsatellites are now known in these respective species, and these could become the source of markers for the application targeted in this invention.

The term "Genotyping" refers to methods to assay which alleles at one or several DNA sequence polymorphisms are present in the analyzed samples. There are several methods to genotype DNA sequence polymorphisms, which are well known in the art and can be applied in the present invention. One commonly used method to genotype large numbers of SNP in parallel relies SNP genotyping arrays. With regards to the bovine, there are at present three favored bovine SNP genotyping arrays, including respectively -3,000 (Golden Gate 3K Bovine array), 7,000 (Bovine LD Bead Chip) 50,000 (Bovine SNP50 Bead Chip) and 700,000 (Bovine HD Bead Chip) SNPs distributed across the genome. All of these are developed and commercialized by Illumina Inc. (http://www.illumina.com/). Other types of arrays and genotyping methods are continuously being developed and improved, but the basic objective remains the same, i.e. determine which alleles are present in the analyzed sample at the interrogated DNA sequence polymorphism. DNA sequencing is the ultimate genotyping technology. It is possible that sequencing will supersede all other genotyping technologies in the future.

The term "quantifying the proportion of the different alleles" refers to determining the relative abundance of each allele at an interrogated DNA sequence polymorphism in the analyzed sample. To achieve this, one needs to use quantitative genotyping methods. There are several methods to quantitatively genotype DNA sequence polymorphisms, which are well known in the art and can be applied in the present invention.

The term "estimating the proportion of DNA contributed by each cow on the farm" refers to methods to determine the composition of the DNA extracted from the milk tank in terms of the relative contribution of each cow, i.e. what proportion of the DNA in the milk tank was contributed by cow 1, 2, 3 , ... n. Provided that the genotype of all cows on the farm are known for all utilized DNA sequence polymorphism, this can be accomplished using a variety of mathematical approaches known in the art. The genotypes of the cows will be of type 11, 12 or 22 for DNA sequence polymorphisms with two alleles herein referred to as 1 and 2, respectively (such as most SNPs), or 11, 12, 13, 22, 23, ... for multiallelic markers (such as microsatellites). The mathematical approaches that can be used include least squares (as illustrated hereafter in the detailed description of the invention) but also maximum likelihood or Bayesian methods. The SNP genotypes of the individual cows can either be real genotypes, determined using the laboratory procedures referred to above, or can be genotypes that are determined in silico using a procedure that is well known in the art and referred to as "genotype imputation" (Marchini & Howie NRG 11,499, 2010). When using imputation, genotypes can be straightforwardly replaced by genotype probabilities in the mathematical approaches referred to above.

The term "estimating the somatic cell scores of each cow on the farm" refers to the conversion of the relative amounts of DNA contributed by each cow in relative somatic cells contributed by each cow or, better, the actual concentration of somatic cell numbers for each cow. The first requires knowledge of the amount of milk contributed by each cow to the tank milk. This information is generally available as present-day milking instrumentation automatically collects the amount of milk obtained from each cow. The second requires information about the number of somatic cells in the tank milk. This information is often determined by the dairy factories that are collecting the milk as milk pricing is influenced by this parameter.

A further embodiment of the invention relates to a method for indirectly determining the number of somatic cells in the milk of individual sheep or goat, the method comprising the steps of obtaining a sample of milk from the farm's milk tank, -

a. extracting DNA from said sample,

b. genotyping said DNA for a collection of DNA sequence polymorphisms,

c. quantifying the proportion of the different alleles for each of the said DNA sequence polymorphisms in said DNA,

d. estimating the proportion of DNA contributed by each sheep/goat on the farm to tank's milk,

e. estimating the somatic cell scores of each sheep/goat on the farm from said proportion of DNA contributed.

Applying the invention to sheep or goat requires the use of DNA sequence polymorphisms for these species. They are of the same type as fort cattle, i.e. including SNPs, micro- and mini-satellites, indels and CNV. Such DNA sequence polymorphisms are already known in both species and their numbers will continue to increase as more research is conducted in these species. At the time of writing, a SNP genotyping array was already commercially available from Illumina (Ovine SNP50 BeadChip) for sheep.

**Detailed description of the invention**

**MATERIALS AND METHODS**

***Method***. Assume a dairy farm with *n* cows. Assume that the volume of milk contributed by cow *i* to the milk tank is known (as it is often in reality) and equals $v_i$. Assume that the somatic cell counts per liter of milk contributed to the tank by cow *i* is $c_i$. Assume that all cows on the farm have been genotyped for an array interrogating m SNPs. Assume that $g_{ij}$ is an indicator variable for the genotype of cow *i* for SNP *j* (taking a value of 1 for genotype 11, 0.5 for genotype 12, and 0 for genotype 22). Assume that a sample of DNA extracted from the milk tank has been genotyped with the same array, and that the estimated frequency of allele "1" of SNP *j* in the milk sample is $f_j$. The proportion of somatic cells contributed by cow *i* to the tank, $pc_i$, can be estimated from a set of *m* linear equation of the form:

$$f_j = \sum_{i=1}^{n} pc_i \times g_{ij} + \varepsilon_j$$

by minimizing

$$SSE = \sum_{j=1}^{n} \varepsilon_j$$

From the obtained $pc_i$, values one can then determine the somatic cell counts per liter of cow *i* relative to the rest of the herd as -

$$rc_i = pc_i / pv_i = c_i / \dot{c}$$

in which $pv_i$ is the known proportion of the tank's volume contributed by cow *i*, and $\dot{c}$ is the average somatic cell count per liter in the herd. If $\dot{c}$ is known (it can be measured in the milk tank), the absolute somatic cell counts per liter for cow *i* can be computed from $rc_i$.

***Simulations.*** We simulated farms with 25, 100 and 500 cows. The cows' daily milk production (liter) was assumed normally distributed with mean 30 l and standard deviation 0.2. Somatic cell counts per liter were assumed to be proportional (x $8 \times 10^6$) to a chi-squared distribution (2df). We assumed the use of SNP arrays with 3,000, 50,000 or 700,000 SNPs corresponding closely to presently available commercial products. Cows were assumed to have genotypes for each SNP (in practice, missing values would be filled in by imputation). Estimates of SNP allele frequencies ($0<f<1$) in the tank milk were assumed normally distributed around the true frequency with standard error of 0.05.

***Real data.*** We collected a sample of milk from a tank containing known quantities of milk (mean: 29.5 liter; SD: 5.3 liter) contributed the same day by 20 cows that had been previously genotyped using either of two custom-made ~50K Illumina arrays (Charlier et al., 2008). Individual somatic cell counts were determined on the same day for each cow using a Fossomatic FC instrument (Foss, Hilerod, Denmark) DNA was extracted from the milk sample using standard procedures and genotyped with the USDA ~50K Illumina array (Matukumalli et al., 2009). Estimates of B-allele frequencies (corresponding to $f_i$ in Method) were directly obtained from the Beadstudio software package (http://www.illumina.com/). We also had SNP genotypes for 20 cows from the same farm that did not contribute milk to the tank.

Statistical analyses were conducted in R.

**RESULTS**

***Simulated data***. Figure 1 is showing representative examples confronting actual and estimated SCC. Predictions were very accurate with coefficient of determination > 0.98 (i.e. $r^2$ = proportion of the variance of true SCC accounted for by predictions) with 3K or more SNPs for 25 cows, 50K or more SNPs for 100 cows and 700K SNPs for 500 cows. Predictors appeared unbiased under all tested conditions. Thus, our simulations indicated that bulk genotyping of tank milk could be effective for SCC monitoring of individual cows, including identification of cows with increased SCC indicative of subclinical mastitis.

Figure 2 shows representative examples of frequency distribution of the statistical significance (*-log(p))* of the $pc_i$'s for cows that did contribute milk to the tank, evaluated with a standard t-test. For ~80% of the cows, p-values were ≤ 0.0001 when using arrays interrogating ≥ 3K SNPs for herds with 25 cows, ≥ 50K SNPs for herds with 100 cows. As expected p-values were strongly and inversely correlated with the proportion of SCC contributed by a given cow to the milk tank (data not shown). By comparison, (figure 2B) the frequency distribution of corresponding p-values for cows that did not contribute milk to tank (added one at the time in the model), largely followed the uniform distribution expected under the null hypothesis, despite a slight excess of low p-values (two-fold excess of p-values < 0.01, including three-fold excess of p-values < 0.001).

***Real data.*** We selected 8,696 SNPs that were (i) interrogated by the three utilized SNP panels, and (ii) for which genotype information was complete for all 40 analyzed cows (20 that did contribute milk to the tank and 20 that did not). Figure 3 shows the distribution of minor allele frequencies in the real data set for these 8,696 SNPs. The distribution was fairly uniform and comparable to the simulated SNP sets. Figure 4 shows the relation between measured SCC and SSC estimated from the SNP allele frequencies in the pooled milk sample. The correlation was highly significant ($r^2 = 0.587$; $p<0.0001$), yet lower than in equivalent simulated datasets. P-values associated with $pc_i$'s were $\leq 10^{-4}$ for all cows, except the one with lowest SCC (10,000/ml; p = 0.006). However, when adding cows that did not contribute milk to the tank one-by-one in the linear model, the p-value of the corresponding regression coefficients ($pc_i$) was $\leq$ 0.0025 (i.e. a Bonferroni-corrected 5% threshold) for approximately one third of the cows (Figure 5).

## DISCUSSION

In this work, we present a novel approach for determining SCC for individual cows by genotyping a sample of milk from the milk tank, i.e. without having to perform a separate measurement for each animal. The method presupposes that individual SNP genotypes are available for each cow. This will increasingly correspond to reality as (i) genotyping costs continue to drop, and (ii) applications of genomic selection extend to cows.

The method proved effective using both simulated and real data. Not unexpectedly, for comparable scenarios in terms of number of cows and SNPs, the coefficient of determination ($r^2$) was considerably better with the simulated than with the real data. Several factors could contribute to this discrepancy, including (i) inaccuracies in counting somatic cells in real samples, (ii) underestimation of the inaccuracies in estimating SNP allele frequencies in the simulation, (iii) absence of linkage disequilibrium between simulated SNPs leading to an overestimation of the informativeness of the simulated SNP sets, (iv) relatedness between real but not simulated cows. For all these reasons, application of the proposed method in the field would certainly benefit from maximizing the number of utilized SNPs to compensate for propagation of inaccuracies from various sources.

The SNP genotypes of the individual cows could either be real genotypes, or - assuming that the SNP panel used on the tank milk is not identical to that used on the cows - imputed genotypes. Methods for genotype imputation are well established and particularly effective in livestock as close relatives are often genotyped, allowing exploitation of Mendelian and within-family linkage information in addition to population-wide linkage disequilibrium information. Alternatively, it might be possible to impute allelic frequencies for the tank milk at missing SNP positions from the allelic frequencies measured at flanking markers and from the known linkage disequilibrium structure in the herd of interest. The impact of genotype (frequency) imputation on the precision of the proposed method will have to be evaluated.

In Belgium, SCC is typically evaluated -10 times per year at a cost of -25 Euros/cow/year. In a farm with 100 cows, this amounts to -2,500 Euros/year. At the present price, this corresponds to > 25 analyses with a 50K SNP array, and - 10 analyses with a 700K SNP array. Thus the argument can be made that - even today - the proposed methodology is price-competitive.

Analyzing DNA extracted from tank milk will become increasingly useful and cost-effective as other applications are becoming available. Monitoring the milk's microbiome, including detection of putative pathogens, by means of high-throughput sequencing of - for instance - 16S rRNA amplicons is certainly one application that is virtually immediately practical.

We explored the possibility to determine whether specific cows did or not contribute milk to the tank. This might for instance be useful to monitor the respect of exclusion of milk from treated cows to the tank. Related applications have previously been evaluated in the context of human genetics (Homer et al., 2008). In this study, we used the statistical significance of the cow-specific $pc_i$ regression coefficients (measured using a standard t-test) as an indicator of the presence or absence of milk of a specific cow in the pool. While specificity and sensitivity appeared satisfactory with the simulated data, this was not the case with real data. The reasons underlying this discrepancy could be multiple and are presently being examined.

In summary, we herein describe the principles and demonstrate the feasibility of a novel approach to determine SCC of individual cows that has the potential to be a valuable addition to the arsenal of methods to control mastitis including subclinical.

## Brief description of the figures

**Figure 1: Simulated data.** Representative examples of the relationship between actual SCC and predictions based on SNP genotyping of a sample from the tank milk. Evaluated scenarios consider 25, 100 and 500 cows genotyped for 3,000, 50,000 or 700,000 SNPs. $R^2$ corresponds to the coefficient of determination.

**Figure 2: Simulated data. (A)** Frequency distribution of the statistical significance (t-test) of the $pc_i$ regression coefficients for (i) simulated cows that did not contribute milk to the tank added one-by-one in a linear model including all the cows that did contribute milk to the tank (black bars), (ii) simulated cows that did contribute milk to the tank and use of a linear model where all cows included in the model contributed milk to the tank (grey bars). **(B)** Expected (null hypothesis, hashed) and observed (black) distribution of log(1/p) values for simulated cows that did not contribute milk to the tank. In general expected and observed distributions match closely, with however a slight excess of observed low p-values.

**Figure 3: Real data.** Distribution of minor allele frequency amongst 40 analyzed cows for 8,696 SNPs utilized to estimate SCC of individual cows from the SNP allele frequencies in the tank milk.

**Figure 4: Real data (A)** Relationship between measured SCC and SCC estimated by SNP genotyping milk from a tank including milk from 20 cows.

**Figure 5: Real data.** Distribution of the statistical significance (log(1/p) values estimated using a standard t-test) of the regression coefficient corresponding to (i) the 20 cows that did contribute milk to the tank (Hatched=In/in: linear model including the 20 cows that did contribute milk to the tank; checker board patern=IN/in+out: linear model including all 40 cows), and (ii) the 20 cows that did not contribute milk to the tank (Grey=Out/in: linear model including the 20 cows that did contribute milk to the tank and one that did not; black=Out/in+out: linear model including all 40 cows).

## References

Charlier C, Coppieters W, Rollin F, Desmecht D, Agerholm JS, Cambisano N, Carta E, Dardano S, Dive M, Fasquelle C, Frennet JC, Hanset R, Hubin X, Jorgensen C, Karim L, Kent M, Harvey K, Pearce BR, Simon P, Tama N, Nie H, Vandeputte S, Lien S, Longeri M, Fredholm M, Harvey RJ, Georges M. (2008) Highly effective SNP-based association mapping and management of recessive defects in livestock. Nat Genet. 40:449-454.

Homer N, Szelinger S, Redman M, Duggan D, Tembe W, Muehling J, Pearson JV, Stephan DA, Nelson SF, Craig DW.(2008) Resolving individuals contributing trace amounts of DNA to highly complex mixtures using high-density SNP genotyping microarrays. PLoS Genet 4:e1000167.

Hoogeveen, H, Huijps K, Lam TJGM (2011) Economic aspects of mastitis: new developments. New Zealand Veterinary Journal 59:16-23.

Hovinen M, Pyörälä S (2011) Udder health of dairy cows in automatic milking. J Dairy Sci 94:547-562.

Matukumalli LK, Lawley CT, Schnabel RD, Taylor JF, Allan MF, Heaton MP, O'Connell J, Moore SS, Smith TP, Sonstegard TS, Van Tassell CP (2009) Development and characterization of a high density SNP genotyping assay for cattle. PLoS One 4: e5350.


## Claims

**1.** A method for indirectly quantifying the number of somatic cells in the milk of an individual animal, the method comprising the steps of:

a. obtaining a sample of milk from the farm's milk tank,
b. extracting DNA from said sample,
c. genotyping said DNA for a collection of DNA sequence polymorphisms,
d. quantifying the proportion of the different alleles for each of the said DNA sequence polymorphisms in said DNA,
e. estimating the proportion of DNA contributed by each animal on the farm to tank's milk,
f. estimating the somatic cell scores of each animal on the farm from said proportion of DNA contributed.

**2.** The method of claim 1 in which the animal is a Bos taurus, Bos indicus or hybrid cow.

**3.** The method of claim 1 in which the animal is a Ovis aries sheep.

**4.** The method of claim 1 in which the animal is a Capra a hircus goat.

**5.** The method of claim 2 in which genotyping is performed using the Illumina Golden Gate 3K Bovine array.

**6.** The method of claim 2 in which genotyping is performed using the Illumina Bovine LD Bead Chip array.

**7.** The method of claim 2 in which genotyping is performed using the Illumina Bovine SNP50 Bead Chip array.

**8.** The method of claim 2 in which genotyping is performed using the Illumina Bovine HD Bead Chip array.

**9.** The method of claim 3 in which genotyping is performed using the Illumina Ovine 50K BeadChip array.

## Patentansprüche

1. Verfahren für die indirekte quantitative Bestimmung der somatischen Zellzahl in der Milch eines Einzeltiers, wobei das Verfahren die folgenden Schritte umfasst:

   a. Entnehmen einer Milchprobe aus dem Milchtank des landwirtschaftlichen Betriebs,
   b. Extrahieren von DNA aus der Probe,
   c. Genotypisierung der DNA für eine Sammlung von DNA-Sequenzpolymorphismen,
   d. quantitatives Bestimmen des Anteils der unterschiedlichen Allele von jedem der DNA-Sequenzpolymorphismen in der DNA,
   e. Abschätzen des Anteils der DNA, die jedes Tier auf dem landwirtschaftlichen Betrieb zu der Tankmilch beiträgt,
   f. Abschätzen der somatischen Zellwerte jedes Tiers auf dem landwirtschaftlichen Betrieb aufgrund des Anteils der beigetragenen DNA.

2. Verfahren nach Anspruch 1, wobei es sich bei dem Tier um eine Bostaurus-, Bos-indicus- oder Hybridkuh handelt.

3. Verfahren nach Anspruch 1, wobei es sich bei dem Tier um ein Ovisaries-Schaf handelt.

4. Verfahren nach Anspruch 1, wobei es sich bei dem Tier um eine Capra-a.-hircus-Ziege handelt.

5. Verfahren nach Anspruch 2, wobei die Genotypisierung unter Verwendung des Illumina Golden Gate 3K Bovine-Array erfolgt.

6. Verfahren nach Anspruch 2, wobei die Genotypisierung unter Verwendung des Illumina Bovine LD Bead Chip-Array erfolgt.

7. Verfahren nach Anspruch 2, wobei die Genotypisierung unter Verwendung des Illumina Bovine SNP50 Bead Chip-Array erfolgt.

8. Verfahren nach Anspruch 2, wobei die Genotypisierung unter Verwendung des Illumina Bovine HD Bead Chip-Array erfolgt.

9. Verfahren nach Anspruch 3, wobei die Genotypisierung unter Verwendung des Illumina Ovine 50K BeadChip-Array erfolgt.

## Revendications

1. Méthode de quantification indirecte du nombre de cellules somatiques dans le lait d'un animal individuel, la méthode comprenant les étapes

   a. d'obtention d'un échantillon de lait à partir de la cuve de lait de l'exploitation,
   b. d'extraction d'ADN à partir dudit échantillon,
   c. de génotypage dudit ADN pour une collection de polymorphismes de séquences d'ADN,
   d. de quantification de la proportion des différents allèles pour chacun desdits polymorphismes de séquences d'ADN dans ledit ADN,
   e. d'estimation de la proportion d'ADN apporté par chaque animal de l'exploitation au lait de la cuve,
   f. d'estimation des scores de cellules somatiques de chaque animal de l'exploitation à partir de ladite proportion d'ADN apporté.

2. Méthode selon la revendication 1, dans laquelle l'animal est *Bos taurus, Bos indicus* ou une vache hybride.

3. Méthode selon la revendication 1, dans laquelle l'animal est une brebis, *Ovis aries.*

4. Méthode selon la revendication 1, dans laquelle l'animal est une chèvre, *Capra hircus.*

5. Méthode selon la revendication 2, dans laquelle le génotypage est effectué en utilisant la puce Golden Gate 3K Bovine d'Illumina.

6. Méthode selon la revendication 2, dans laquelle le génotypage est effectué en utilisant la puce Bovine LD Bead Chip d'Illumina.

7. Méthode selon la revendication 2, dans laquelle le génotypage est effectué en utilisant la puce Bovine SNP50 Bead Chip d'Illumina.

8. Méthode selon la revendication 2, dans laquelle le génotypage est effectué en utilisant la puce Bovine HD Bead Chip d'Illumina.

9. Méthode selon la revendication 3, dans laquelle le génotypage est effectué en utilisant la puce Ovine 50K Bead Chip d'Illumina.

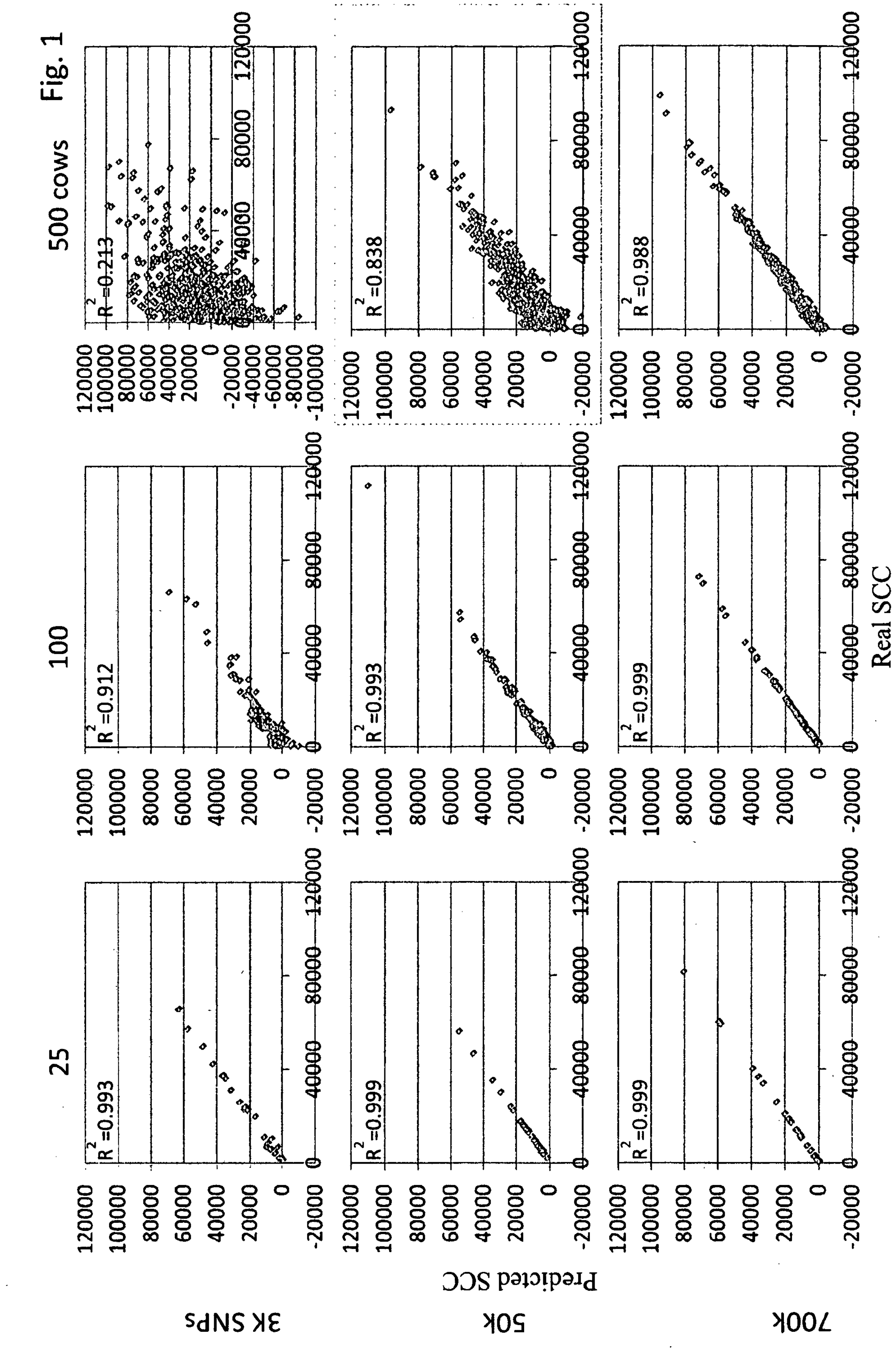
25
100
500 cows
3K SNPs
50K
700K
Predicted SCC
Real SCC
R2 =0.993
R2 =0.912
R2 =0.213
R2 =0.999
R2 =0.993
R2 =0.838
R2 =0.999
R2 =0.999
R2 =0.988

Fig. 1

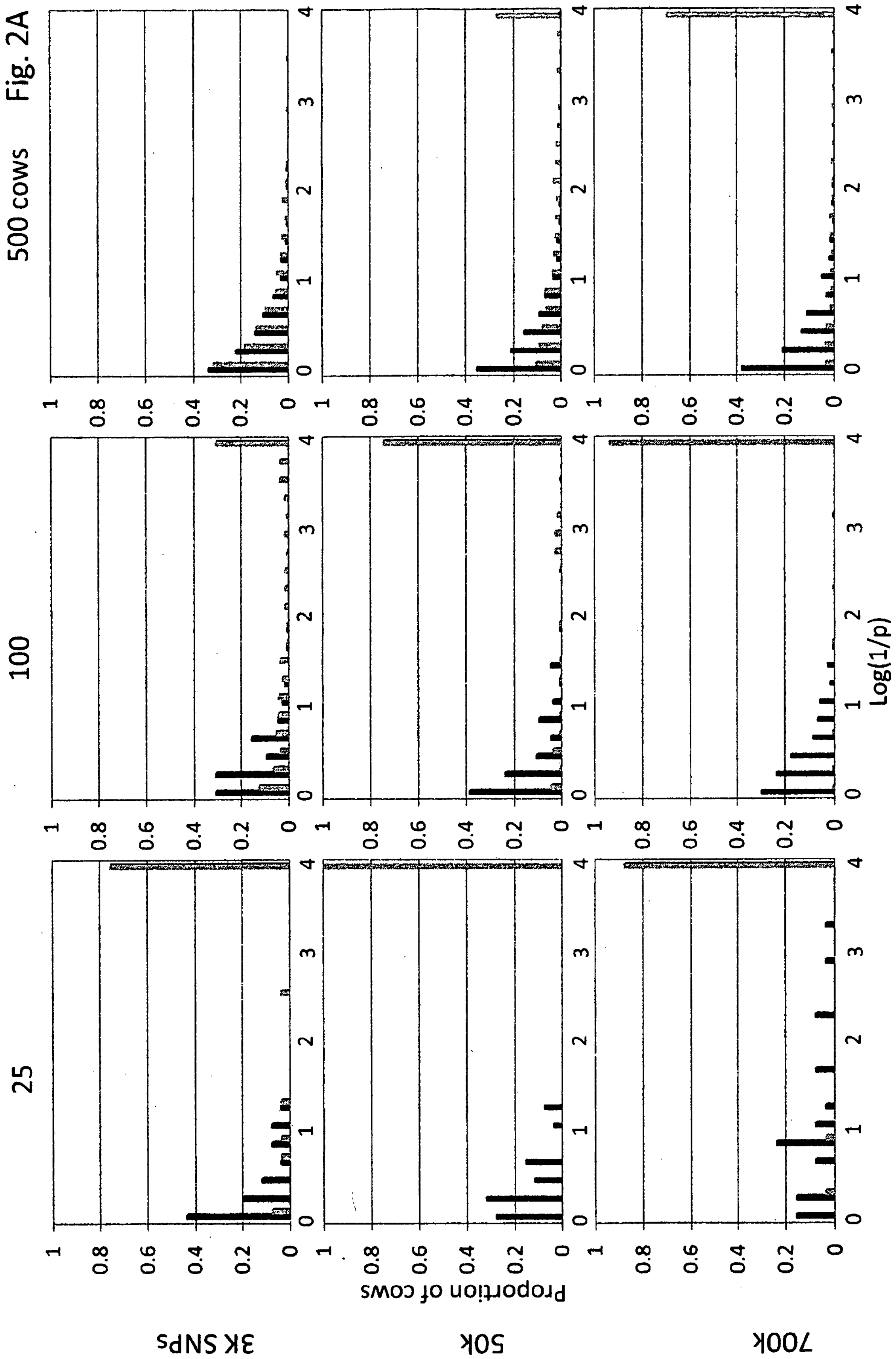
Fig. 2A
500 cows
100
25
3K SNPs
50K
700K
Proportion of cows
Log(1/p)
0
0.2
0.4
0.6
0.8
1
0
1
2
3
4

Fig. 2B

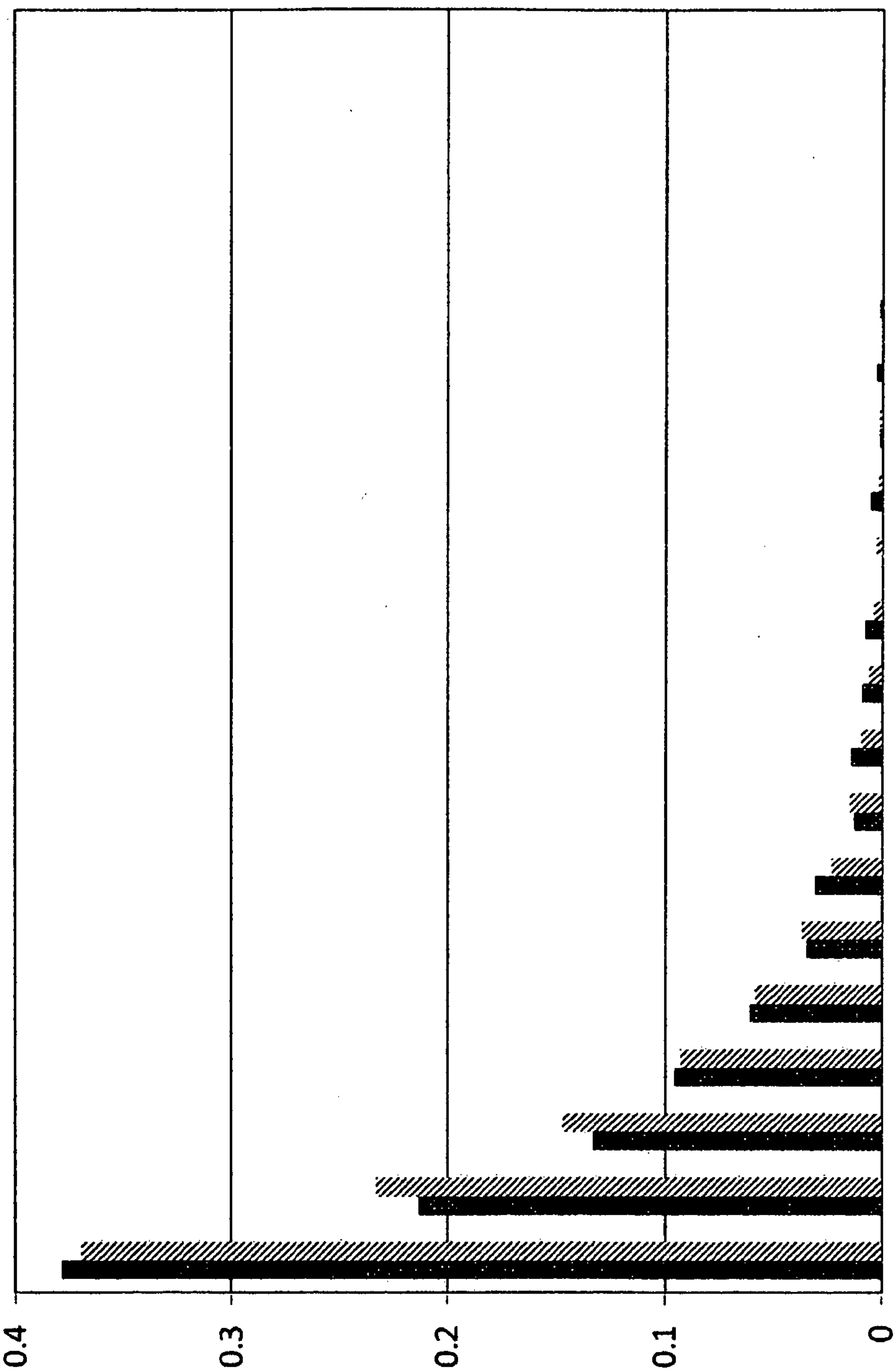

Fig. 3

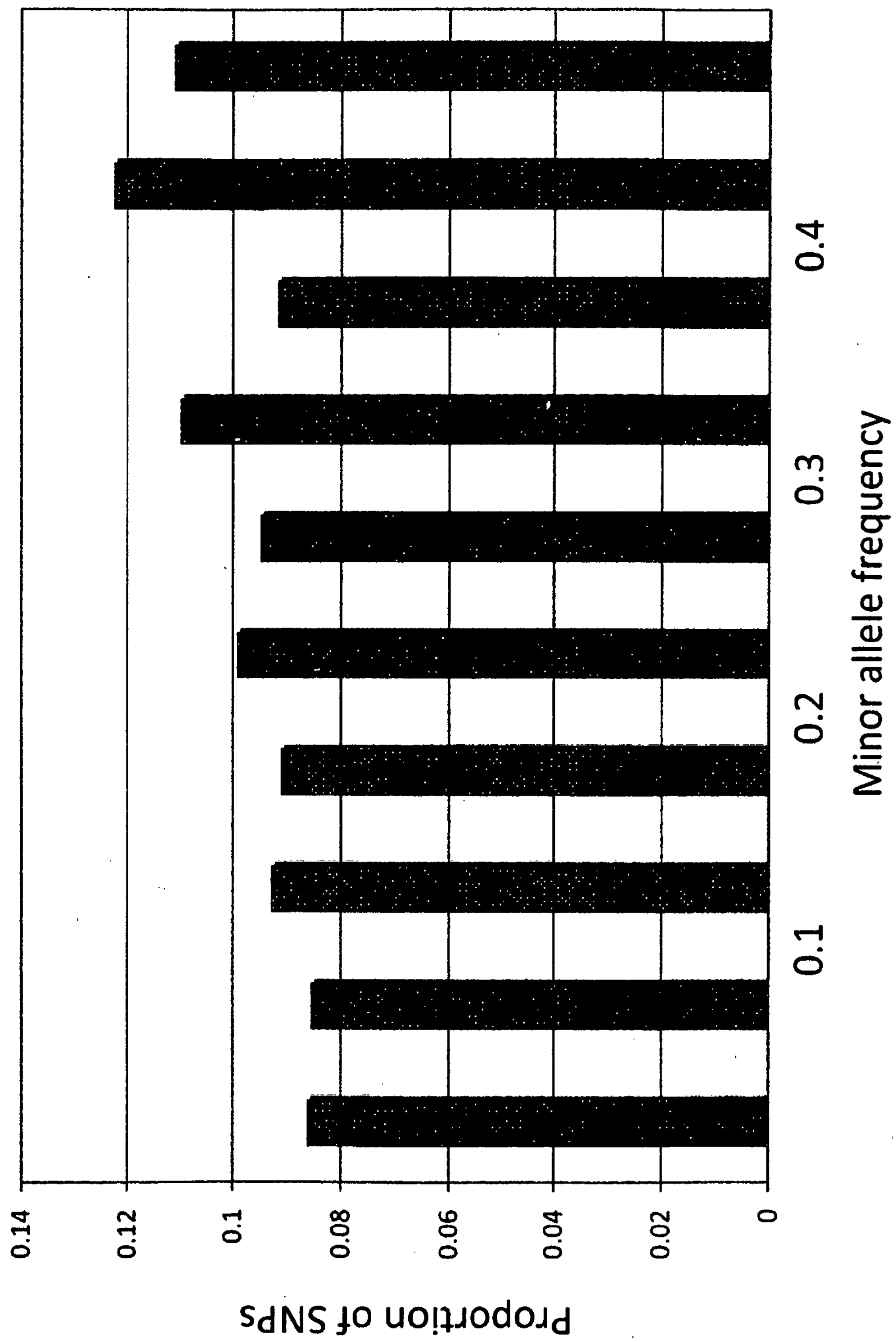
0.14
0.12
0.1
0.08
0.06
0.04
0.02
0
Proportion of SNPs
0.1
0.2
0.3
0.4
Minor allele frequency

Fig. 4

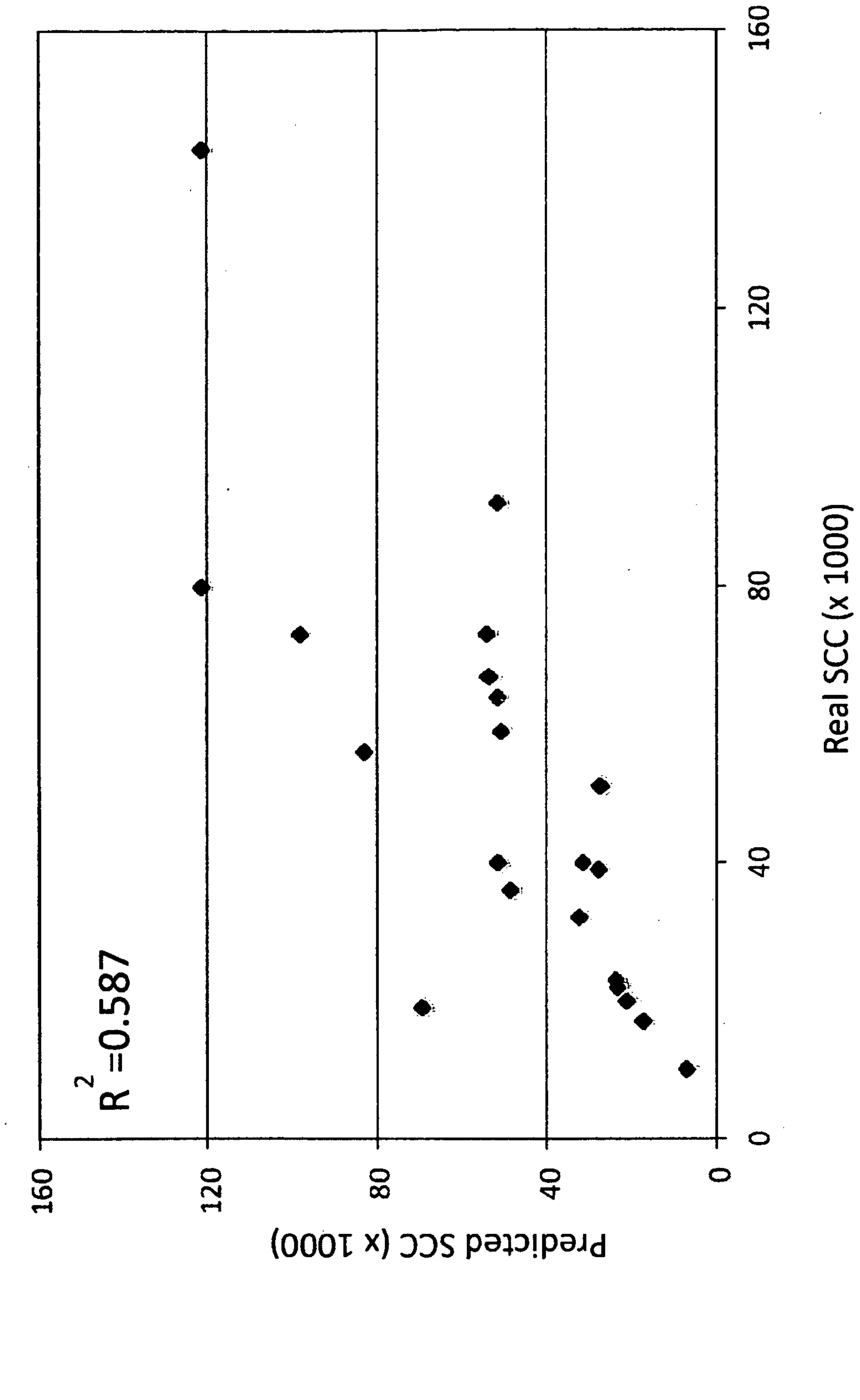
$R^2$ =0.587
Predicted SCC (x 1000)
0
40
80
120
160
Real SCC (x 1000)
0
40
80
120
160

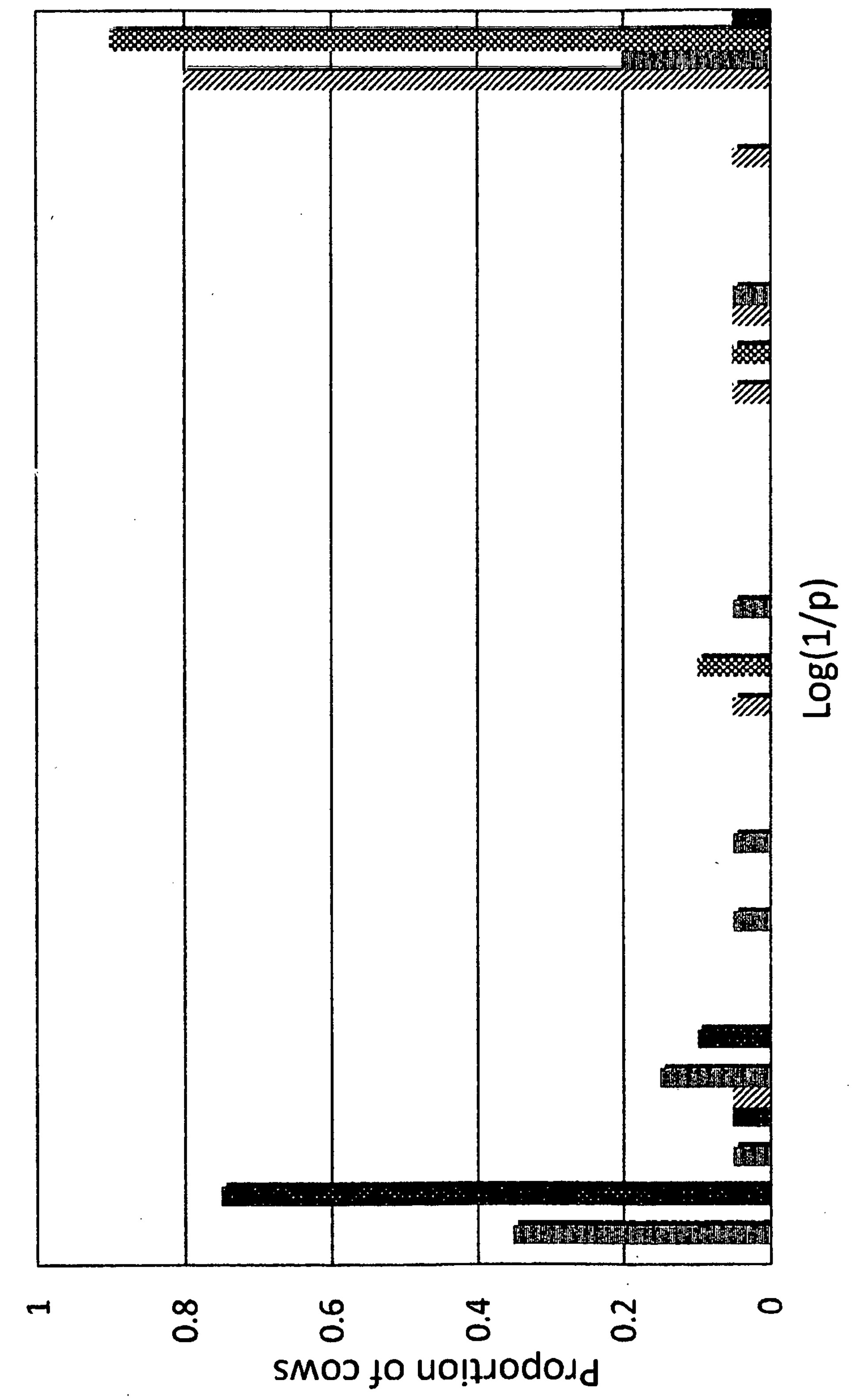
In/In
Out/In
In/In+Out
Out/In +out
Proportion of cows
1
0.8
0.6
0.4
0.2
0
Log(1/p)

Suppl. Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SAMBROOK, J. ; RUSSELL, D.W.** Molecular Cloning: A Laboratory Manual. Cold Spring Harbor Laboratory Press, 2001, 1.31-1.38
- **SHARMA, R.C. et al.** A rapid procedure for isolation of RNA-free genomic DNA from mammalian cells. *BioTechniques,* 1993, vol. 14, 176-178
- **CHARLIER C ; COPPIETERS W ; ROLLIN F ; DESMECHT D ; AGERHOLM JS ; CAMBISANO N ; CARTA E ; DARDANO S ; DIVE M ; FASQUELLE C.** Highly effective SNP-based association mapping and management of recessive defects in livestock. *Nat Genet,* 2008, vol. 40, 449-454
- **HOMER N ; SZELINGER S ; REDMAN M ; DUGGAN D ; TEMBE W ; MUEHLING J ; PEARSON JV ; STEPHAN DA ; NELSON SF ; CRAIG DW.** Resolving individuals contributing trace amounts of DNA to highly complex mixtures using high-density SNP genotyping microarrays. *PLoS Genet,* 2008, vol. 4, e1000167
- **HOOGEVEEN, H ; HUIJPS K ; LAM TJGM.** Economic aspects of mastitis: new developments. *New Zealand Veterinary Journal,* 2011, vol. 59, 16-23
- **HOVINEN M ; PYÖRÄLÄ S.** Udder health of dairy cows in automatic milking. *J Dairy Sci,* 2011, vol. 94, 547-562
- **MATUKUMALLI LK ; LAWLEY CT ; SCHNABEL RD ; TAYLOR JF ; ALLAN MF ; HEATON MP ; O'CONNELL J ; MOORE SS ; SMITH TP ; SONSTEGARD TS.** Development and characterization of a high density SNP genotyping assay for cattle. *PLoS One,* 2009, vol. 4, e5350